# EUROPEAN PATENT SPECIFICATION

(11) **EP 1 956 886 B1**
(45) Date of publication and mention of the grant of the patent: **18.08.2010**
(21) Application number: 06835638.5
(22) Date of filing: 27.11.2006
(51) Int. Cl.: A01H 3/04, A01G 7/06, A01N 43/90

(54) **IMPROVED ORCHID CULTURING METHOD**
VERBESSERTES ORCHIDEENKULTIVIERUNGSVERFAHREN
PROCEDE DE CULTURE D'ORCHIDEES AMELIORE

(30) Priority: 25.11.2005 EP 05077680; 02.12.2005 NL 1030581
(43) Date of publication of application: 20.08.2008
(73) Proprietor: Bakker, Joost Petrus Jacobus, 2377 CA Oude Wetering (NL)
(72) Inventor: Bakker, Joost Petrus Jacobus, 2377 CA Oude Wetering (NL)
(74) Representative: Hatzmann, Martin
(86) International application number: PCT/NL2006/000593
(87) International publication number: WO 2007/061296

(56) References cited:
- EP-A1- 0 753 257
- US-B1- 6 168 952
- C. J. GOH: "Hormonal regulation of flowering in a sympodial orchid hybrid Dendrobium louisae" NEW PHYTOLOGIST, vol. 82, no. 2, 1979, pages 375-380, XP002425564 CAMBRIDGE, GB
- GOH CHONG JIN: "Flowering of Orchids" PHILIPPINE ORCHID REVIEW, [Online] 1 June 1986 (1986-06-01), XP002425565 Retrieved from the Internet: URL:http://philorchidsociety.org/index.php ?option=com_content&task=view&id=47&Itemid =38> [retrieved on 2007-03-19]

## Description

The invention relates to an improved method for the culturing of orchids, more specifically *Phalaenopsis.*

The sales of potted orchids has increased steadily over the last 30 years. In 2000, wholesale orchid sales were approximately $100,000,000, of which about 75% was accounted for by *Phalaenopsis* (Griesbach, R.J., 2002. Development of Phalaenopsis Orchids for the Mass Market. p. 458-465, In: Janick J. and Whipkey A. (eds), Trends in new crops and new uses, ASHS Press, Alexandra, VA).

*Phalaenopsis,* or the moth or butterfly orchid, is a genus of the family of Orchidiaceae, which contains at least 60 different species. However, in the recent past many varieties and hybrids have been produced for commercial purposes, resulting in a large assortment of pot flowers. An important group of these hybrids is formed by plants of the genus *Doritaenopsis,* which arise from a first cross between a *Phalaenopsis* plant and a plant of the genus *Doritus.* Very many subvarieties, created through further crosses with *Phalaenopsis* or *Doritaenopsis* are known in the meantime. Comprised in the term "orchids of the genus *Phalaenopsis* and *Doritaenopsis"* are thus also all hybrids which have been created by a cross with one of these.

All orchids have these five basic features :
- the presence of a column
- the flower is bilaterally symmetrical
- the pollen is glued together into the pollinia, a mass of waxy pollen on filaments.
- the seeds are microscopically small, lacking endosperm (food reserves).
- the seeds can, under natural circumstances, only germinate in symbiosis with specialized fungi. Under artificial circumstances, however, germination is possible "in vitro" on sterile substrates or agar in specialized laboratories.

*Phalaenopsis* (as well as *Doritaenopsis*) shows a monopodial growth habit. An erect growing rhizome produces from the top one or two alternate, thick and fleshy, elleptical leaves a year. The older, basal leaves drop off at the same rate. The plant retains in this way four to five leaves. They have no pseudobulbs. The raceme (flowering stem or stalk, spike or inflorescence) appears from the stem between the leaves. *Phalaenopsis* typically produces a single raceme, which consists of up to 25 flowers. The species and hybrids of *Phalaenopsis* that bloom in later winter or early spring require a period of about six weeks of exposure from 25°C-30°C (culture) to 15°C-20°C (induction) to trigger the emergence of the inflorescence (Report "Cultivation Guidelines Phalaenopsis Pot Plant. Anthura B.V. and Bureau IMAC Bleiswijk B.V., http://www.anthura.nl). There is an absolute requirement for the presence of light while plants are exposed to the proper temperatures for spiking (= emergence of the inflorescence). Although the plants are marketed year round, winter is an important selling season. This means that for the plants which have to be sold during winter, the cold induction has to be performed during the summer months. This means that the greenhouses need to be cooled down to at least 20 °C, which increases the costs of growing the plants.

It is already known in the prior art that treatment with hormones, especially cytokinins, can induce growth of shoots (JP9233962 and Nayak, N.R. et al., 1998, In Vitro Cell. Dev. Biol. - Plant 34:185-188) or flowering in orchids, as well *in vitro* (Kostenyuk, I. et al., 1999, Plant Cell Reports, 19:1-5; Kim, T.-J., et al., 1999, J. Kor. Soc. Hort. Sci. 40:619-622; Duan, J.-X. and Yazawa S., 1995a, Plant Cell, Tissue and Organ Culture, 43:71-74) as *in vivo* (Yoneda, K. and Momose, H., 1990, Bull. Coll. Agr. & Vet. Med., Nihon Univ., 47:71-74; and Duan, J.-X. and Yazawa, S., 1995b, Acta Horticulturae 397:103-110). See also Goh, C. J., 1979, New Physiologist, 82:375-380.

It is desirable to generate more inflorescences on the same plant, since this would yield more flowers per plant, and thus would increase its value. Depending on the length and temperature of the cold induction *Phal;aenopsis* plants normally induce one or two flower stalks, and exceptionally three, and very exceptionally four flower stalks.

In the above-mentioned prior art, the presence of multiple flower stalks is what can be expected according to the normal existing growing practice: little number of plants shows one or two additional flower stalks. Induction of multiple flower stalks by the hormone treatment has only been described with the *in vitro* treatment of shoots with cytokinin (Duan, J.-X. en Yazawa, S, 1995b, *supra*)*,* wherein the authors, who concomitantly also describe *in vivo* experiments in these latter plants, did not report an increase in the number of flower stalks. Further, the art is silent about the effect of a combined hormone treatment and cold induction.

Thus, there is still need for a method to induce multiple raceme formation in orchids.

### SUMMARY OF THE INVENTION

The present inventors now have found a method to increase the number of inflorescences in an orchid of the genus *Phalaenopsis* or *Doritaenopsis* by administration of a cytokinin to said orchid, before or during the period of exposure of said orchid to a cold period for inducing flowering. Said cytokinin is preferably 6-BAP (6-benzylaminopurine), which can be applied in a concentration in the range of 2 mg to 30 mg per plant, preferably 5 mg to 26 mg per plant, more preferably 10 mg to 24 mg per plant, most preferably about 20 mg per plant. In aqueous spraying solution, this would result in a range of 100 ppm to 1500 ppm, preferably 250 ppm to 1300 ppm, more preferably 500 ppm to 1200 ppm, most preferably 600-1000 ppm.

A preferred embodiment of the invention is wherein the administration of the cytokinin is done by spraying a solution comprising said cytokinin. Additionally, for better uptake of the cytokinin, the orchid is kept in the dark for a period of 6 to 14 hours after spraying. A similar effect, enhanced uptake of cytokinin, can be achieved by increasing the relative humidity of the air in the greenhouse. These measures can be taken separately, dependant on the climatic conditions inside and outside the greenhouse. Also for better uptake the spraying solution can additional comprise a wetting agent, preferably Zipper®, and/or a co-solvent, preferably DMSO.

A further preferred embodiment comprises the administration of a rooting hormone, preferably NAA to the orchid (uptake of NAA takes place both at the leaves and at the roots). Administration of the rooting hormone preferably takes place at transfer of the young plants to pots and can be performed to about 4 weeks previous to the cold induction period.

A following embodiment of the invention is an orchid of the genus *Phalaenopsis* or *Doritaenopsis,* which has more than four inflorescences. Also a group of orchids of the genus *Phalaenopsis* or *Doritaenopsis* of one and the same variety of 1000 or more, having an average number of inflorescences of more than 2.1, preferably more than 2.5, more preferably more than 2.9, is included in the scope of the present invention. Also contemplated are the use of a cytokinin to induce the forming of adventitious inflorescences in an orchid and the use of a cytokinin to abolish or diminish the need for a cold period to induce inflorescences.

### DESCRIPTION OF THE DRAWINGS

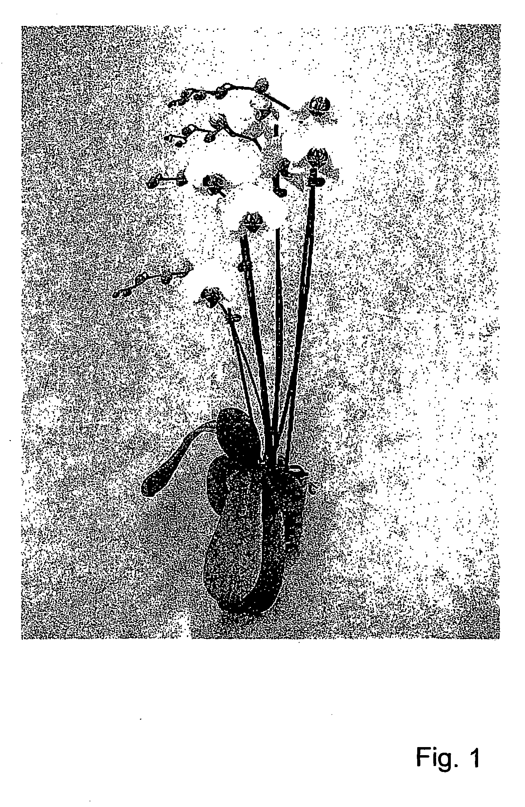

Fig. 1 shows a plant of the species *Phalaenopsis Hybrid* showing four flower stalks.

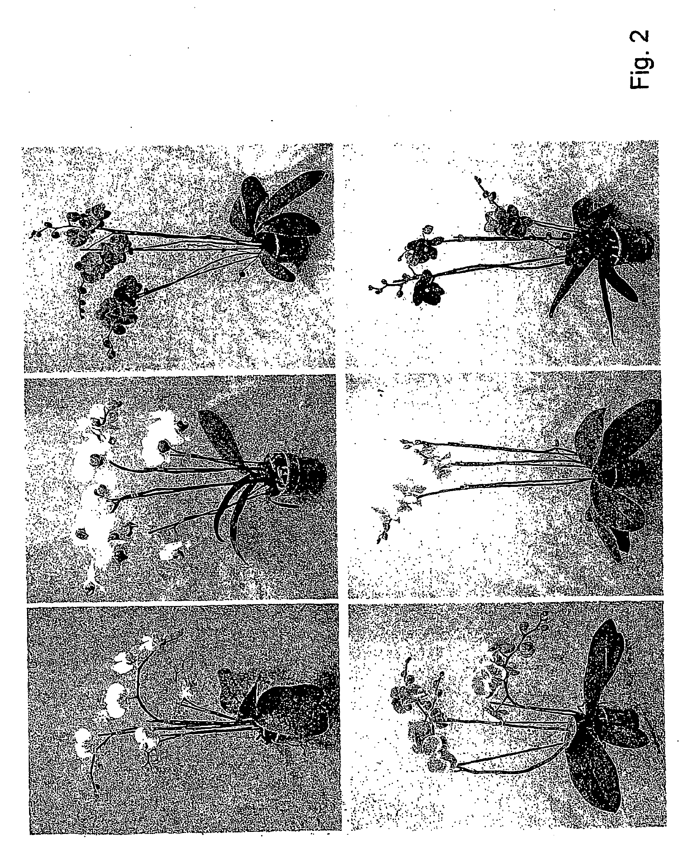

Fig. 2 shows results of example 1.

### DETAILED DESCRIPTION OF THE INVENTION

Cytokinins have the effect of increasing the mitosis (cell division) of shoot and buds. Some cytokinins increase the size or number of the plant cells and e.g. make leaves larger. The cell differentiation can be controlled (e.g. for production of calii). Growth of roots is inhibited. Cytokinins are adenine derivatives and kinetin can e.g. be synthesised from heating nucleic acid. Known cytokinins are: 2iP N6-(3-methylbut-2-enyl)adenine, Kinetin (6-furfurylaminopurine), Benzyladenine (N6-benzyladenine), BA or BAP (6-benzylaminopurine), Zeatin, Adenine sulfate (2C₅H₅N₅. H₂SO₄) and Thidiazurone (1-phenyl-3-(1,2,3-tiadiazole-5-yl)urea). Cytokinins are heavily used in orchid production during tissue culture, to induce shoot regeneration. Further, it has been demonstrated that cytokinins can be used to induce keiki's in orchids (a keiki is a small plant which grows from one of the nodes along the (flower-bearing) stem). Usually these keiki's are introduced by applying cytokinins (commercially available as 'keiki paste', e.g. KeikiGrow from Plant Hormones Canada) after flowering. However, it has been reported (Wang, Y-T.: Medium, Nutrition and Flower Induction in Potting Blooming Orchids, ASHS-2000 Symposium, http://primera.tamu.edu/orchids/wang.htm) that application of cytokinin (BA or a combination of PBA and GA), whereas this was shown to trigger spiking - by application to the mature pseudobulbs, but not the developing ones - in Dendrobium, was unable to induce off-season spiking in *Phalaenopsis.* One possible reason for this failure is that the development of the inflorescence in *Dendrobium* occurs through so-called pseudo-bulbs, which are absent in *Phalaenopsis.*

The inventors, however, have now found that application of a cytokinin yields the emergence of adventitious inflorescences in orchids, especially orchids with a monopodial growth, preferably orchids that do not have pseudobulbs, and more preferably *Phalaenopsis* or *Doritaenopsis.* Upon cytokinin application it appears that sometimes four or more additional flower stalks appear. These additional spikes, in contrast to so-called secondary flower stalks, are about similar to the original one and can also bear a large amount of flowers (under optimal growing conditions).

The cultivation and production of potted orchids in general and specifically *Phalaenopsis,* nowadays has taken an enormous leap due to the availability of tissue engineering for large scale production of clones. Since the 1970's several tissue culture protocols specific for *Phalaenopsis* have been developed. These tissue explants (mostly explants from the flower stalk) are regenerated to plantlets in large facilities, which have capacities of producing millions of plantlets a year. It is also possible to cultivate the orchids from seed, which would yield a pottable plant after about 15 months. The grower obtains these small plantlets and cultivates them further (for at least another 20 weeks) before induction of the florescence is initiated. After about 6 weeks at lower temperature, it will take about another 16 weeks to grow the plant, now bearing a flower stalk, into a sellable product, which is a plant which has just started flowering. The cultivation from plantlet to flowering plant is almost exclusively done in greenhouses because of the requirements of temperature control.

Normally the plants are held at a temperature of about 25 °C to about 30 °C, preferably about 27 °C. When flowering needs to be induced the temperature is lowered to about 15 °C to about 20 °C, preferably about 18 °C. In summer, in temperate climates, this would approximate the outside temperature, which means that this cold period can be achieved by only terminating the previous heating to 27 °C, whereupon the temperature in the greenhouse would drop to the outside temperature. However, since this outside temperature is far from constant and - in hot summers - would easily be above the desired temperature of max 20 °C, many breeders opt for active cooling.

It has now appeared possible to minimise the active cooling and still be able to induce the inflorescence by applying cytokinin. This treatment not only minimises the cold induction, but it appeared that more flower stalks are formed than with the normal cold induction. Whereas plants which have been cold induced normally would develop only one flower stalk (or two), treatment with cytokinin normally would give two flower stalks and in many cases more than two.

The number of flower stalks is related principally to the age of the plant (in fact the number of leaves and - defined thereby - the number of eyes from which primary flower stalks can grow). The top three leaves of the plant are too juvenile to be of influence on the formation of flower stalks, but every pair of leaves beneath them can form the basis of one flower stalk. The older the plant, i.e. the more (still or not any more present) leaves, the more possibilities the orchid has for the formation of flower stalks. In principle, thus, more than two (to maximally four) flower stalks could be generated from very old plants, but because this necessitates the use of old plants, it is not an economically cost-effective way to increase the number of flower stalks.

Further, typically plants of the genus *Phalaenopsis* and *Doritaenopsis* fit the above description. Some exceptions exist of plants, which standardly give a multiplicity of flower stalks and yet should be classified within one of both genera. These are: *P*. *amabilis* and *P.* "Anthura Gold". For the purpose of this application, these are regarded as atypical plants, while the plants which normally would give one or sometimes two flower stalks in conventional culture are indicated as typical plants of the genus *Phalaenopsis* and *Doritaenopsis.*

Preferably synthetic cytokinins are used, most preferably BAP because of its low cost: purification of naturally occurring cytokinins is time consuming and expensive. Further it is know that synthetic cytokinins are not toxic in the concentrations used, nor for plants nor for animals, including humans (TAP Report for 6-benzyladenine, January 2004, at http://www.ams.usda.gov/nop/NationalList/6BenzyladenineFinalnoCBI.pdf). Synthetic cytokinins according to the present invention are substances that show a cytokinin activity and are not to be found as such in nature, notably in plants (e.g. thidiazuron, benzyladenine and 6-benzylaminopurine).

Application of the cytokinin can be performed in any conventional way, but in greenhouses with many plants, it is preferable to apply the cytokinin by spraying with an aqueous solution. The concentration of the cytokinin to be sprayed can vary, but should be in the range of 100 ppm to 1500 ppm, preferably 250 ppm to 1300ppm, more preferably 500 ppm to 1200 ppm, most preferably 600-1000 ppm.

Spraying is done with an amount of about 100 liters of spraying solution for an amount of approximately 20.000 plants. Thus, each plant will receive about 1/200 liter, or 5 ml of spraying solution. Spraying can be performed using standard spraying equipment, such as 5- 25 Bar pressure pump with single or multiple nozzles as commonly used for application of fungicides and/or pesticides. Such equipment is commercially available. This equipment preferably nebulises the spraying solution, in order that both upper and under surface of the leaves come into contact with the spraying solution.

When recalculating the above mention preferred concentrations in amounts of cytokinin applied to individual plants, it would result in 2 to 30 mg per plant, preferably 5 mg to 26 mg per plant, more preferably 10 mg to 24 mg per plant, most preferably about 20 mg per plant.

Next to administration via spraying solution, the cytokinin can also be applied according to other ways known in the art, e.g. through powder coating, electro-spray, through normal techniques for watering (e.g. ebb- and flood-technique) or through direct application on or next to the plant, e.g. in the form of pastes, patches or strips, or plant sticks with slow release of the cytokinin to the substrate.

To increase the uptake of the cytokinin by the plants, additives, such as co-solvents or wetting agents, can be added to the cytokinin solution. One co-solvent which is preferably used in the invention is DMSO, which preferably is added to the spraying solution to a final concentration of 1 liter per 100 liter spraying solution. As wetting agent the commercially available Zipper® (an organically modified trisiloxane, obtainable from Asepta, Delft., The Netherlands) can be used in a final concentration of 10-500 ml per 100 liter spraying solution, preferably 100 ml/liter.

Generally all means that enhance the uptake of the cytokinin by the plant can be used. Next to the above discussed wetting agent Zipper®, other compounds that decrease the surface tension of the liquid are equally applicable. Also agents that enhance the penetration of the cytokinin, such as DMSO (Broome, O.C., Zimmerman, R.H., (1976) J. Amer. Soc. Hort. Sci. 101:28-30) can be useful in the present invention.

To further increase uptake of the cytokinin by the plants, the plants can be covered from the light source, directly after spraying, e.g. by a plastic tent. By thus inducing an artificial night environment the stomata of the plants will open, which facilitates uptake. Further, because of the low light level, evaporation of the spraying solution will minimalise, which means that the sprayed solution will stay in contact with plants for a longer time. Further, in the case of a tent, ventilation will be reduced, which decreases evaporation and increases the relative humidity of the air, which also enhances uptake.

Preferably, covering of the plants only lasts for about 4-48 hours, since orchids need light to grow. After removal of the cover, plants are allowed to continue to grow at the low temperature conditions for another three to seven weeks, preferably about five weeks. After that, the temperature regimen should be returned to the high temperature, i.e. preferably between about 18 °C and 25 °C, to develop the plants to marketable early flowering plants for another 8-23 weeks. If desired, plants can be 'harvested' already earlier and transported or marketed.

Administration of the cytokinin thus is preferably done at the start or just before the cold induction period. However, it is also possible to have the administration done plenty of time before (maximally 10 weeks before, but preferably 1, 2 or 3 weeks before) or shortly after (maximally 5 weeks after) the cold induction period.

Growing conditions for the plants throughout the whole greenhouse stay are the same, except for the above discussed variations in light and temperature. Preferably this means that the plants are potted in pots with a diameter of 10-20 cm, with a growing medium of bark, coconut, Styrofoam, sphagnum moss and turf. Preferably they are stored on table-high racks, which facilitates handling. Light intensity on the surface of the pot is 3000-15000 lux (see Schapendonk, A.H.C.M., "Belichting Phalaenopsis", PT-Project 12170, productverslag, Productschap Tuinbouw). Normal daylight is maintained, but preferably in wintertime a lights on:lights off regime of 14:10 hours is used. Watering is preferably automated and is taken care of by a sprinkler system or any other spraying system, an ebb and flow system, via gutters running along the racks, or by submerging the pots in a water basin Nutrients and/or fertiliser are added to the water on a regular basis (EC 0,5 - 2, NPK 20-20-20). See also ook "Cultivation Guidelines Phalaenopsis Pot Plants, *supra.*

A preferred embodiment of the invention is the additional application of hormones which promote root growth, such as commercially available rooting hormone products. To ensure optimal uptake of water and nutrients, a well-developed root system is essential, especially when more than one inflorescence need to be supported. Therefore, it is preferred to add rooting hormone substances to the water and nutrients to induce extra root growth to be able to support the extra inflorescences. Preferably, the rooting hormone is given when transplanting the pottable plants until 4 weeks prior to cooling for induction.

### EXAMPLE

In June 2004 three groups of *Phalaenopsis* plants have been induced 6 months after potting. For testing 100 plants were treated and 100 non-treated controls were used per variety. Both groups of plants were cultured as described above.Induction of spiking started at week 28 by spraying a solution of 1000ppm 6-BAP (BAP-10, Plantwise, Louisville, Kentucky USA), 0.1% Zipper, 1%DMSO] to the treated plants, while the controls did not receive this treatment. The results in Table 1 show the number of inflorescences per plant for the different testgroups.

**Table 1**

| Variety | Average stemcount untreated | Average stemcount treated |
|---|---|---|
| Dutch Lady | 1,58 | 3,84 |
| Maliby Leopard | 1,51 | 3,12 |
| Anthura Malaga | 1,23 | 3,61 |
| Lippe Flair 344 | 1,32 | 3,38 |
| Golden Treasure | 1,44 | 2,97 |
| Brother John Red Delight | 1,13 | 3,20 |

From this experiemnt it appears that groups of orchids of at least 5 plants per variety, or alternative 10, 20, 30, 40, 50, 100 or 1000 plants, can be produced with a method as described above, which have on average more than 2 inflorescences. To discriminate such a group from a random group of five or more control plants of one variety, it can be stated that the group of treated plants has at least an average of 2.1 or more, preferably 2.5 or more and more preferably 2.9 or more flowering stalks. These racemes can support flowering flowers or not.

## Claims

1. Method to increase the number of inflorescences in a typical plant of the genus *Phalaenopsis* or *Doritaenopsis* by administration of a cytokinin to said plant, before or during the exposure of said plant to a cold period for induction of flowering.

2. Method according to claim 1, wherein said cytokinin is a synthetic cytokinin.

3. Method according to claim 2, wherein said cytokinin is 6-BAP (6-benzylaminopurine).

4. Method according to claims 1, 2 or 3 wherein the amount of administered cytokinin per plant is in the range of 2 mg to 30 mg per plant.

5. Method according to claim 4, wherein the amount of administered cytokinin per plant is in the range of 5 mg to 26 mg per plant.

6. Method according to claim 4, wherein the amount of administered cytokinin per plant is in the range of 10 mg to 24 mg per plant.

7. Method according to claim 4, wherein the amount of administered cytokinin per plant is about 20 mg per plant.

8. Method according to one of the previous claims, wherein the concentration of the cytokinin in aqueous solution is in the range of 100 ppm to 1500 ppm.

9. Method according to claim 8, wherein the concentration of the cytokinin in aqueous solution is 250 ppm to 1300 ppm.

10. Method according to claim 8, wherein the concentration of the cytokinin in aqueous solution is 500 ppm to 1200 ppm.

11. Method according to claim 8, wherein the concentration of the cytokinin in aqueous solution is 600-1000 ppm.

12. Method according to any of the previous claims, wherein the administration of the cytokinin is done by spraying a solution comprising said cytokinin.

13. Method according to claim 12, wherein the orchid after spraying is kept in the dark for a period of 6 to 14 hours.

14. Method according to claim 12, wherein the spraying solution additionally comprises a wetting agent and/or a co-solvent.

15. Method according to claim 14, wherein the wetting agent is Zipper®.

16. Method according to any of the previous claims wherein a rooting hormone is applied to the roots of the orchid prior to the administration of the cytokinin.

17. Method for producing a **typical plant of the genus *Phalaenopsis* or** ***Doritaenopsis*** with more than two inflorescences by treating said plant with a method according to any of the conclusions 1-16.

18. Method for producing a group of **typical plants of the genus *Phalaenopsis* or *Doritaenopsis*** of 5 or more plants with an average number of flowering stalks of 2.1 or more by treatment of said group of plants with a method according to any of the conclusions 1-16.

19. Method of claim 18, wherein said group has an average number of flowering stalks of 2.5 or more.

20. Method according to claim 18, wherein said group has an average number of flowerings talks of 2.9 or more.

21. Group of typical plants of the genus *Phalaenopsis* or *Doritaenopsis* of the same variety of 1000 or more plants, which have an average amount of flowering stalks of 2.1 or more, **wherein said plants are produced by a method according to any of claims 18-20.**

22. Group according to claim 21, wherein said group has an average amount of flower stalks of 2.5 or more.

23. Group according to claim 22, wherein said group has an average amount of flower stalks of 2.9 or more.

24. **Typical plant** of the genus *Phalaenopsis* or *Doritaenopsis,* which has more than four inflorescences, **wherein said plant is produced by a method according to claim 17.**

25. Use of a cytokinin to induce the forming of adventitious inflorescences in a typical plant of the genus *Phalaenopsis* or *Doritaenopsis,* when applying the method of claim 1.

26. **Typical plant** of the genus *Phalaenopsis* or *Doritaenopsis* according to claim 24 or belonging to a group according to any of claims 21-23, that comprises a synthetic cytokinin.

## Patentansprüche

1. Verfahren zur Vermehrung der Anzahl an Blütenständen in einer typischen Pflanze der Gattung *Phalaenopsis* oder *Doritaenopsis* durch Verabreichung von einem Cytokinin zu der Pflanze, bevor oder während die Pflanze zur Induktion der Blütezeit einer kalten Periode ausgesetzt wird.

2. Verfahren gemäß Anspruch 1, wobei das Cytokinin ein synthetisches Cytokinin ist.

3. Verfahren gemäß Anspruch 2, wobei das Cytokinin 6-BAP (6-Benzylaminopurin) ist.

4. Verfahren gemäß der Ansprüche 1, 2 oder 3, wobei der Anteil von verabreichtem Cytokinin pro Pflanze im Bereich von 2 mg bis 30 mg pro Pflanze liegt.

5. Verfahren gemäß Anspruch 4, wobei der Anteil von verabreichtem Cytokinin pro Pflanze im Bereich von 5 mg bis 26 mg pro Pflanze liegt.

6. Verfahren gemäß Anspruch 4, wobei der Anteil von verabreichtem Cytokinin pro Pflanze im Bereich von 10 mg bis 24 mg pro Pflanze liegt.

7. Verfahren gemäß Anspruch 4, wobei der Anteil von verabreichtem Cytokinin pro Pflanze etwa 20 mg pro Pflanze ist.

8. Verfahren gemäß einem der vorangehenden Ansprüche, wobei die Konzentration des Cytokinins in wässriger Lösung im Bereich von 100 ppm bis 1500 ppm ist.

9. Verfahren gemäß Anspruch 8, wobei die Konzentration des Cytokinins in wässriger Lösung 250 ppm bis 1300 ppm.

10. Verfahren gemäß Anspruch 8, wobei die Konzentration des Cytokinins in wässriger Lösung 500 ppm bis 1200 ppm ist.

11. Verfahren gemäß Anspruch 8, wobei die Konzentration des Cytokinins in wässriger Lösung 600-1000 ppm ist.

12. Verfahren gemäß einem der vorangehenden Ansprüche, wobei die Verabreichung des Cytokinins durch Sprühen einer Lösung enthaltend das Cytokinin erfolgt.

13. Verfahren gemäß Anspruch 12, wobei die Orchidee nach dem Sprühen für eine Dauer von 6 bis 14 Stunden im Dunkeln aufbewahrt wird.

14. Verfahren gemäß Anspruch 12, wobei die Sprühlösung zusätzlich ein Befeuchtungsmittel und/oder ein Co-Lösungsmittel enthält.

15. Verfahren gemäß Anspruch 14, wobei das Befeuchtungsmittel Zipper® ist.

16. Verfahren gemäß einem der vorangehenden Ansprüche, wobei ein Wurzelhormon zu den Wurzeln der Orchidee vor der Verabreichung des Cytokinins appliziert wird.

17. Verfahren zur Herstellung einer typischen Pflanze der Gattung *Phalaenopsis* oder *Doritaenopsis* mit mehr als zwei Blütenständen durch Behandlung der Pflanze mit einem Verfahren gemäß einer der Aussagen 1-16.

18. Verfahren zur Herstellung einer Gruppe von typischen Pflanzen der Gattung *Phalaenopsis* oder *Doritaenopsis* von 5 oder mehr Pflanzen mit einer durchschnittlichen Anzahl an Blütenstängeln von 2.1 oder mehr durch Behandlung der Gruppe von Pflanzen mit einem Verfahren gemäß einer der Aussagen 1-16.

19. Verfahren gemäß Anspruch 18, wobei die Gruppe eine durchschnittliche Anzahl an Blütenstängeln von 2.5 oder mehr hat.

20. Verfahren gemäß Anspruch 18, wobei die Gruppe eine durchschnittliche Anzahl an Blütenstängeln von 2.9 oder mehr hat.

21. Gruppe von typischen Pflanzen der Gattung *Phalaenopsis* oder *Doritaenopsis* der gleichen Art von 1000 oder mehr Pflanzen, die eine durchschnittliche Anzahl an Blütenstängeln von 2.1 oder mehr haben, wobei die Pflanzen durch ein Verfahren gemäß einem der Ansprüche 18-20 hergestellt sind.

22. Gruppe gemäß Anspruch 21, wobei die Gruppe eine durchschnittliche Anzahl an Blütenstängeln von 2.5 oder mehr hat.

23. Gruppe gemäß Anspruch 22, wobei die Gruppe eine durchschnittliche Anzahl an Blütenstängeln von 2.9 oder mehr hat.

24. Typische Pflanze der Gattung *Phalaenopsis* oder *Doritaenopsis,* die mehr als 4 Blütenstände hat, wobei die Pflanze durch ein Verfahren gemäß Anspruch 17 hergestellt ist.

25. Verwendung eines Cytokinins zum Induzieren der Bildung von adventiven Blütenständen in einer typischen Pflanze der Gattung *Phalaenopsis* oder *Doritaenopsis* bei Anwendung des Verfahrens von Anspruch 1.

26. Typische Pflanze der Gattung *Phalaenopsis* oder *Doritaenopsis* gemäß Anspruch 24 oder zugehörend zu einer Gruppe gemäß einem der Ansprüche 21-23, die ein synthetisches Cytokinin enthält.

## Revendications

1. Procédé permettant d'augmenter le nombre d'inflorescences dans une plante type du genre *Phalaenopsis* ou *Doritaenopsis* par l'administration d'une cytokinine à ladite plante, avant ou pendant l'exposition de ladite plante à une période de froid destinée à induire de la floraison.

2. Procédé selon la revendication 1, dans lequel ladite cytokinine est une cytokinine synthétique.

3. Procédé selon la revendication 2, dans lequel ladite cytokinine est la 6-BAP (6-benzylaminopurine).

4. Procédé selon les revendications 1, 2 ou 3, dans lequel la quantité de cytokinine administrée par plante se situe dans la plage de 2 mg à 30 mg par plante.

5. Procédé selon la revendication 4, dans lequel la quantité de cytokinine administrée par plante se situe dans la plage de 5 mg à 26 mg par plante.

6. Procédé selon la revendication 4, dans lequel la quantité de cytokinine administrée par plante se situe dans la plage de 10 mg à 24 mg par plante.

7. Procédé selon la revendication 4, dans lequel la quantité de cytokinine administrée par plante est d'environ 20 mg par plante.

8. Procédé selon l'une quelconque des revendications précédentes, dans lequel la concentration de la cytokinine en solution aqueuse se situe dans la plage de 100 ppm à 1500 ppm.

9. Procédé selon la revendication 8, dans lequel la concentration de la cytokinine en solution aqueuse est de 250 ppm à 1300 ppm.

10. Procédé selon la revendication 8, dans lequel la concentration de la cytokinine en solution aqueuse est de 500 ppm à 1200 ppm.

11. Procédé selon la revendication 8, dans lequel la concentration de la cytokinine en solution aqueuse est de 600 à 1000 ppm.

12. Procédé selon l'une quelconque des revendications précédentes, dans lequel l'administration de la cytokinine est réalisée par pulvérisation d'une solution comprenant ladite cytokinine.

13. Procédé selon la revendication 12, dans lequel l'orchidée après la pulvérisation est gardée dans l'obscurité pendant une période de 6 à 14 heures.

14. Procédé selon la revendication 12, dans lequel la solution à pulvériser comprend en outre un agent de mouillage et/ou un cosolvant.

15. Procédé selon la revendication 14, dans lequel l'agent de mouillage est Zipper^{®}.

16. Procédé selon l'une quelconque des revendications précédentes, dans lequel une hormone d'enracinement est appliquée sur les racines de l'orchidée avant l'administration de la cytokinine.

17. Procédé de production d'une plante type du genre *Phalaenopsis* ou *Doritaenopsis* avec plus de deux inflorescences par traitement de ladite plante par un procédé selon l'une quelconque des revendications 1 à 16.

18. Procédé de production d'un groupe de plantes types du genre *Phalaenopsis* ou *Doritaenopsis* de 5 plantes ou plus présentant un nombre moyen de tiges fleuries de 2,1 ou plus par traitement dudit groupe de plantes par un procédé selon l'une quelconque des revendications 1 à 16.

19. Procédé selon la revendication 18, dans lequel ledit groupe présente un nombre moyen de tiges fleuries de 2,5 ou plus.

20. Procédé selon la revendication 18, dans lequel ledit groupe présente un nombre moyen de tiges fleuries de 2,9 ou plus.

21. Groupe de plantes types du genre *Phalaenopsis* ou *Dori taenopsis* de la même variété de 1000 plantes ou plus, qui présentent une quantité moyenne de tiges fleuries de 2,1 ou plus, où lesdites plantes sont produites par un procédé selon l'une quelconque des revendications 18 à 20.

22. Groupe selon la revendication 21, présentant une quantité moyenne de tiges fleuries de 2,5 ou plus.

23. Groupe selon la revendication 22, présentant une quantité moyenne de tiges fleuries de 2,9 ou plus.

24. Plante type du genre *Phalaenopsis* ou *Doritaenopsis,* qui présente plus de quatre inflorescences, ladite plante étant produite par un procédé selon la revendication 17.

25. Utilisation d'une cytokinine pour induire la formation d'inflorescences adventives dans une plante type du genre *Phalaenopsis* ou *Doritaenopsis,* lors de l'application du procédé selon la revendication 1.

26. Plante type du genre *Phalaenopsis* ou *Doritaenopsis* selon la revendication 24 ou appartenant à un groupe selon l'une quelconque des revendications 21 à 23, qui comprend une cytokinine synthétique.
